# EUROPEAN PATENT APPLICATION

(11) **EP 4 699 630 A1**
(43) Date of publication of application: **25.02.2026**
(21) Application number: 24195443.7
(22) Date of filing: 20.08.2024
(51) Int. Cl.: A61M 5/28, A61M 5/32, A61M 5/34

(54) **SAFETY SYRINGE**

(71) Applicant: Hung, Chih-Hua, Hsinchu City 300 (TW)
(72) Inventor: Hung, Chih-Hua, Hsinchu City 300 (TW)
(74) Representative: Straus, Alexander

(57) **Abstract**

The present invention relates to a safety syringe (1) which includes a needle seat (2), a connecting seat (3), an injection barrel (4), a plunger (5), and an outer sleeve (6). The outer sleeve (6) is movably sleeved onto the needle seat (2), the connecting seat (3), the injection barrel (4) and the plunger (5). When the outer sleeve (6) is located at a first position (P1), an abutted portion (65) of the outer sleeve (6) is abutted against an annular wing portion (33) of the connecting seat (3) in a way that the needle seat (2) protrudes out of the front end of the outer sleeve (6). In this status, the plunger (5) is able to be used to draw in and inject liquid medicine. When the outer sleeve (6) is located at a second position (P2), the needle seat (2) and the connecting seat (3) are accommodated in the outer sleeve (6), and a first annular protruding rib (64) is able to push the annular wing portion (33) of the connecting seat (3) such that the outer sleeve (6), the needle seat (2) and the connecting seat (3) are removed together. As a result, the outer sleeve (6), the needle seat (2) and the connecting seat (3) are discardable together, and the injection barrel (4) and the plunger (5) are retainable for medicine adding process.

## Description

### BACKGROUND OF THE INVENTION

### 1. Field of the Invention

The present invention relates to a safety syringe and more particularly, to a syringe having a needle seat and an injection barrel, which are separatable from each other for preventing healthcare workers from being pricked by a needle and the resulting injury or infection after performing injection.

### 2. Description of the Related Art

It is well known that healthcare workers often use syringes to perform medical practices such as injection or blood collection. The used syringe has contacted blood or body fluid of a patient. If the patient contracts a blood-borne disease, there is the pathogen of the disease on the needle. If the used needle is accidentally sticked on the healthcare worker or other people, the pricked person is at the risk of infection. Among this kind of diseases infected by needlestick injury, hepatitis B, hepatitis C and acquired immunodeficiency syndrome (AIDS) pose the greatest risk.

In order to reduce the chance of happening of needlestick injury, safety syringes have the special configuration design for engaging the plunger and the needle seat with each other so that the needle seat together with the needle can be pulled into the injection barrel with the plunger. For example, European Patent Publication No. 1421963 A1 disclosed a safety syringe with a retractable needle. This design can reduce the chance of healthcare workers or medical waste handlers being pricked by used needles. However, after such safety syringe is used, the syringe and the needle cannot be separated from each other, so the needle and the syringe should be discarded together. But the syringe is usually uncontaminated and intact, so discarding the syringe causes material waste, imperceptibly increasing medical costs.

Besides, if it is desired to separate the needle from the syringe, an additional auxiliary apparatus is required for performing the needle detaching process. Alternatively, the injection barrel and the needle may be provided therebetween with a detachable structure, but such design may decrease the stability of the syringe in use, causing the needle to sway. Besides, when detaching the needle, people can be still pricked by the needle without prevention. Therefore, the inventor made effort to think about how to provide a structure enabling the needle and the syringe to be separated from each other, and also preventing healthcare workers after using the syringe from the risk of needlestick injury.

### SUMMARY OF THE INVENTION

In view of the fact that the above-described existing syringes still have many shortcomings in actual implementation and use, the inventor uses rich professional knowledge and years of practical experience thereof to make an improvement, and creates the present invention accordingly.

It is a primary objective of the present invention to provide a safety syringe, which is provided with a detachable structure between a needle and an injection barrel, and the structural design provides improved stability for injection or blood collection, and also complete protection for the used needle during its detachment, thereby increasing after-use safety and reducing material waste.

To attain the above objective, the present invention provides a safety syringe which includes a needle seat, a connecting seat, an injection barrel, a plunger, and an outer sleeve. The needle seat is provided at the rear end thereof with a first axial hole. The connecting seat has an annular wall portion, a second axial hole penetrating through two ends of the annular wall portion, an annular wing portion provided on the outer circumferential surface of the rear end of the annular wall portion, and a stopping portion provided on the inner circumferential surface of the rear end of the annular wall portion. The annular wall portion is sleeved onto the rear end of the needle seat. The second axial hole communicates with the first axial hole of the needle seat. The annular wing portion is formed with a plurality of grooves arranged equidistantly. The injection barrel has a first tube body, an injection portion integrally formed at the front end of the first tube body, and an accommodating opening provided at the rear end of the first tube body. The injection portion is detachably inserted in the first axial hole of the needle seat and the second axial hole of the connecting seat. The plunger is movably inserted in the first tube body of the injection barrel. The outer sleeve has a second tube body, a first opening provided at the front end of the second tube body, a second opening provided at the rear end of the second tube body, a first annular protruding rib located adjacent to the second opening, and an abutted portion provided at the front end of the second tube body. The first annular protruding rib is provided on the inner circumferential surface of the second tube body. The abutted portion extends from the first opening inwardly. The outer sleeve is sleeved onto the injection barrel and movable between a first position and a second position along the axial direction of the injection barrel. When the outer sleeve is located at the first position, the second tube body of the outer sleeve is sleeved onto the first tube body of the injection barrel, the needle seat protrudes out of the first opening of the outer sleeve, and the abutted portion is abutted against the annular wing portion of the connecting seat. When the outer sleeve is located at the second position, the needle seat and the connecting seat are accommodated in the second tube body of the outer sleeve, and the first annular protruding rib is enabled to push the annular wing portion of the connecting seat such that the outer sleeve, the needle seat and the connecting seat are separated from the injection barrel together.

In an embodiment of the present invention, the outer circumferential surface of the rear end of the needle seat has an outer flange.

In an embodiment of the present invention, the inner circumferential surface of the annular wall portion of the connecting seat has an inner flange. The outer flange of the needle seat and the inner flange of the connecting seat are abutted against each other.

In an embodiment of the present invention, the inner circumferential surface of the annular wall portion of the connecting seat has an internal thread. The outer flange of the needle seat and the internal thread of the connecting seat are engaged with each other.

In an embodiment of the present invention, the stopping portion of the connecting seat includes a plurality of positionally limiting protrusions arranged equidistantly. The front end of the first tube body of the injection barrel is formed with a plurality of positioning protrusions arranged equidistantly. When the injection portion is inserted in the first axial hole of the needle seat and the second axial hole of the connecting seat, the plurality of positioning protrusions are jammed in gaps between the plurality of positionally limiting protrusions.

In an embodiment of the present invention, the outer circumferential surface of the annular wall portion of the connecting seat has a plurality of longitudinal outer ribs.

In an embodiment of the present invention, the outer circumferential surface of the first tube body of the injection barrel has a plurality of longitudinal ribs. When the outer sleeve is located at the first position, the first annular protruding rib of the outer sleeve is tightly abutted against the plurality of longitudinal ribs of the injection barrel.

In an embodiment of the present invention, the inner circumferential surface of the second tube body of the outer sleeve further has a second annular protruding rib and a third annular protruding rib. The second annular protruding rib is provided adjacent to the first opening. The third annular protruding rib is provided adjacent to the first annular protruding rib. When the outer sleeve is located at the first position, the annular wing portion of the connecting seat is located between the second annular protruding rib and the abutted portion of the outer sleeve. When the outer sleeve is located at the second position, the annular wing portion of the connecting seat is located between the first annular protruding rib and the third annular protruding rib.

### BRIEF DESCRIPTION OF THE DRAWINGS

FIG. 1 is an exploded view of the structure of the embodiment of the present invention using a connecting seat.
FIG. 2 is an exploded view of the structure of the embodiment of the present invention using another connecting seat.
FIG. 3 is a schematic view of the embodiment of the present invention, showing a first position.
FIG. 4 is a planar view of the appearance of a connecting seat of the embodiment of the present invention.
FIG. 5 is a sectional view taken along the line A-A in FIG.4.
FIG. 6 is a planar view of the appearance of another connecting seat of the embodiment of the present invention.
FIG. 7 is a sectional view taken along the line A-A in FIG. 6.
FIG. 8 is a schematic view of the embodiment of the present invention when drawing medicine.
FIG. 9 is a schematic view of the embodiment of the present invention when injecting medicine.
FIG. 10 is a schematic view of the embodiment of the present invention, showing a second position.
FIG. 11 is a schematic view of the embodiment of the present invention, showing a detachment.
FIG. 12 is a schematic view of the embodiment of the present invention during needle-free medicine providing.

### DETAILED DESCRIPTION OF THE INVENTION

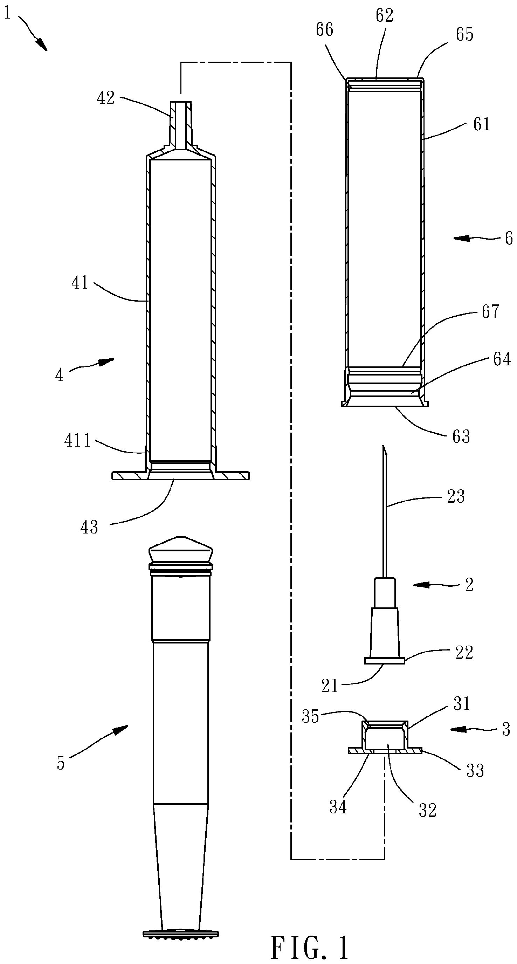

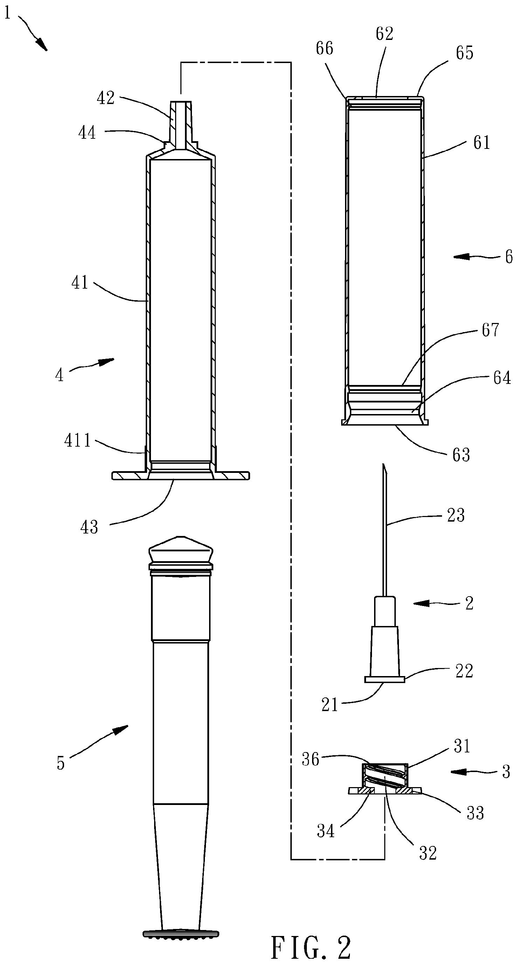

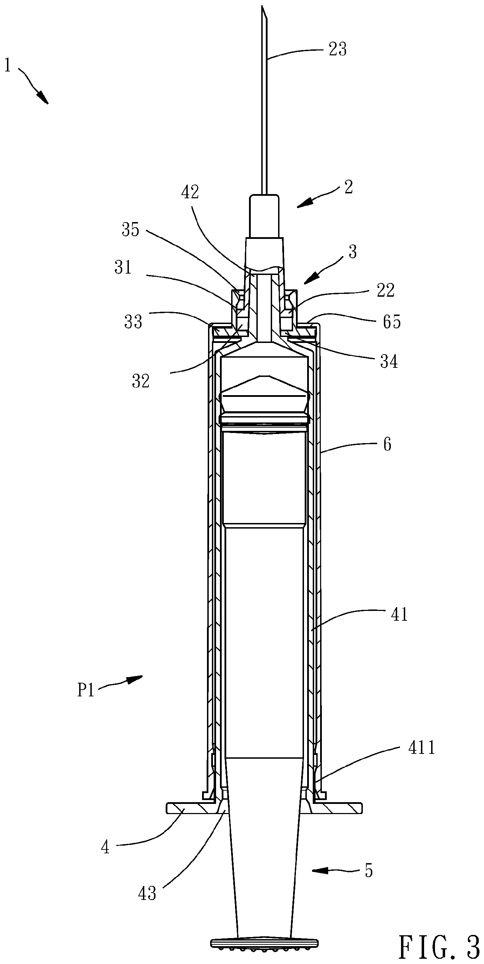

Referring to FIG. 1 to FIG. 3, the safety syringe 1 of the present invention primarily includes a needle seat 2, a connecting seat 3, an injection barrel 4, a plunger 5, and an outer sleeve 6.

The rear end of the needle seat 2 has a first axial hole 21 and an outer flange 22. The front end of the needle seat 2 is adapted for fixing a needle 23.

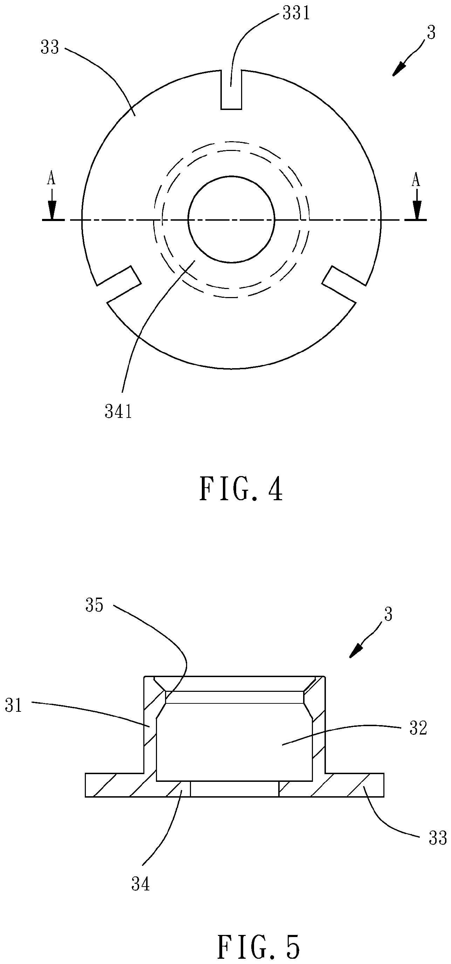

The connecting seat 3 has an annular wall portion 31, a second axial hole 32, an annular wing portion 33, and a stopping portion 34. During assembly, the annular wall portion 31 of the connecting seat 3 is sleeved onto the rear end of the needle seat 2 such that the second axial hole 32 communicates with the first axial hole 21. The inner circumferential surface of the front end of the annular wall portion 31 has an inner flange 35, as shown in FIG. 4 and FIG. 5. In the process that the connecting seat 3 is sleeved onto the needle seat 2, the needle seat 2 is firstly posed at an angle for the outer flange 22 of the rear end thereof to pass a side of the inner flange 35, and then a force is applied to the connecting seat 3 to make the outer flange 22 pass another side of the inner flange 35. In this way, the rear end of the needle seat 2 is accommodated in the annular wall portion 31 of the connecting seat 3 and the second axial hole 32, and the outer flange 22 of the needle seat 2 is in contact with the inner circumferential surface of the annular wall portion 31 of the connecting seat 3, such that the needle seat 2 is fixed in the connecting seat 3, and the inner flange 35 of the connecting seat 3 can prevent the needle seat 2 from separation from the connecting seat 3 through the front end of the second axial hole 32.

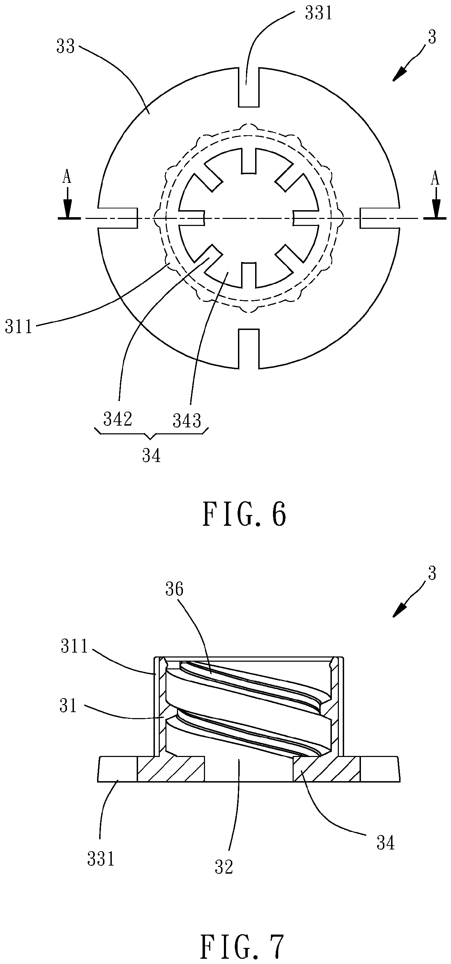

Besides, the inner circumferential surface of the annular wall portion 31 of the connecting seat 3 can be designed with an internal thread 36, and the outer circumferential surface of the annular wall portion 31 has a plurality of longitudinal outer ribs 311 for increasing the structural strength of the annular wall portion 31, as shown in FIG. 6 and FIG. 7. In the process that the connecting seat 3 is sleeved onto the needle seat 2, the needle seat 2 is firstly posed at an angle for the outer flange 22 of the rear end thereof to be engaged to the internal thread 36 at the front end of the annular wall portion 31, and then the outer flange 22 is completely engaged with the internal thread 36 by rotation. In this way, the rear end of the needle seat 2 is accommodated in the annular wall portion 31 of the connecting seat 3 and the second axial hole 32, and the needle seat 2 is fixedly screwed into the connecting seat 3.

For preventing the needle seat 2 from separation from the connecting seat 3 through the rear end of the second axial hole 32, the inner circumferential surface of the rear end of the annular wall portion 31 of the connecting seat 3 is formed with a stopping portion 34, which is an integrally formed annular sheet 341, as shown in FIG. 4. Alternatively, the stopping portion 34 can be designed to include a plurality of positionally limiting protrusions 342 arranged equidistantly, and gaps 343 formed between the positionally limiting protrusions 342, as shown in FIG. 6. Such two types of stopping portion 34 can both stop the needle seat 2 from separation from the connecting seat 3 through the rear end of the second axial hole 32.

In addition, the injection barrel 4 is combined with the connecting seat 3 and the needle seat 2. The injection barrel 4 has a first tube body 41, an injection portion 42, and an accommodating opening 43. The plunger 5 is inserted into the first tube body 41 through the accommodating opening 43. The injection portion 42 protrudes outwardly from the front end of the first tube body 41, and is inserted in the first axial hole 21 of the needle seat 2 and the second axial hole 32 of the connecting seat 3.

In the condition that the stopping portion 34 of the connecting seat 3 is the annular sheet 341, the outer circumferential surface of the injection portion 42 of the injection barrel 4 and the stopping portion 34 of the connecting seat 3 are abutted against each other, such that the injection portion 42 of the injection barrel 4 is fixed in the needle seat 2 and the connecting seat 3. In the condition that the stopping portion 34 of the connecting seat 3 includes the positionally limiting protrusions 342, the first tube body 41 of the injection barrel 4 is provided with a plurality of positioning protrusions 44 arranged equidistantly. The positioning protrusions 44 are connected to both the front end of the first tube body 41 and the outer circumferential surface of the rear end of the injection portion 42. In this way, when the injection portion 42 is inserted in the first axial hole 21 of the needle seat 2 and the second axial hole 32 of the connecting seat 3, the positioning protrusions 44 are jammed in the gaps 343 between the positionally limiting protrusions 342, such that the injection barrel 4 is fixed with improved firmness.

The outer sleeve 6 has a second tube body 61, a first opening 62, a second opening 63, a first annular protruding rib 64, and an abutted portion 65. The first annular protruding rib 64 is provided on the inner circumferential surface of the second tube body 61, and located adjacent to the second opening 63. The abutted portion 65 is provided at the front end of the second tube body 61, and extends from the first opening 62 inwardly. The outer sleeve 6 is sleeved onto the injection barrel 4 through the second opening 63, and moved to a first position P1. At this time, the first annular protruding rib 64 of the outer sleeve 6 is tightly abutted against a plurality of longitudinal ribs 411 of the outer circumferential surface of the first tube body 41. The annular wing portion 33 of the connecting seat 3 is located inside the second tube body 61 and abutted against the abutted portion 65, such that the injection barrel 4 is disabled from separation from the outer sleeve 6 through the first opening 62, and the needle seat 2 and the annular wall portion 31 of the connecting seat 3 protrude out of the first opening 62 of the outer sleeve 6.

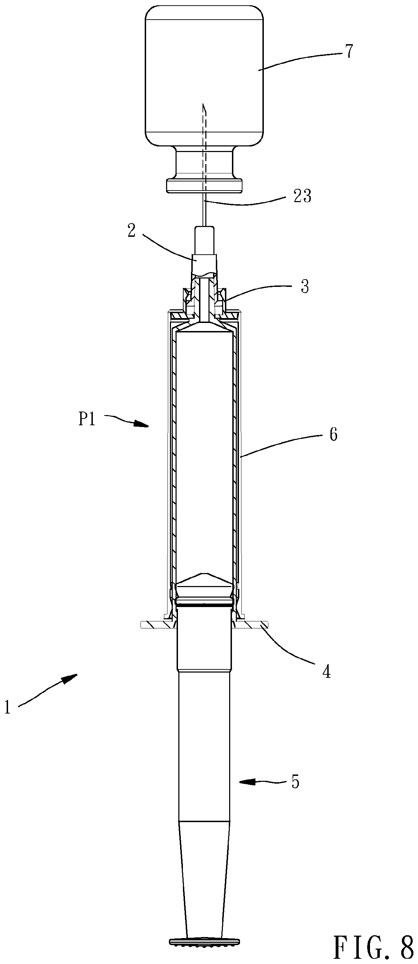

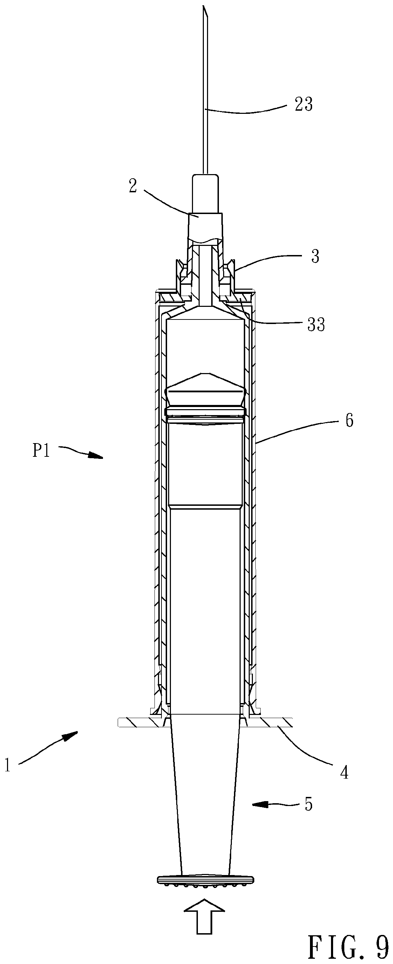

Referring to FIG. 8 and FIG. 9, when the outer sleeve 6 is located at the first position P1, the needle 23 can be inserted into a medicine bottle 7, and the plunger 5 can be pulled to draw the medicine into the injection barrel 4. After that, the needle 23 can be aimed at an injection portion of a patient and prick there, and the plunger 5 can be pushed for injecting the medicine.

Because the annular wing portion 33 of the connecting seat 3 is an annular sheet, in the process that the plunger 5 is pulled or pushed, the thin shape or elasticity of the annular wing portion 33 may cause the needle seat 2 to sway, bringing trouble in use. Therefore, in the present invention the annular wing portion 33 is formed with a plurality of grooves 331 arranged equidistantly. For example, two to four grooves 331 may be provided, as shown in FIG. 4 and FIG. 6. The grooves 331 can increase hardness for the annular wing portion 33, making the needle seat 2 relatively more stable when the plunger 5 is moved, so that the sway is decreased and the convenience for medicine injection or blood collection is improved.

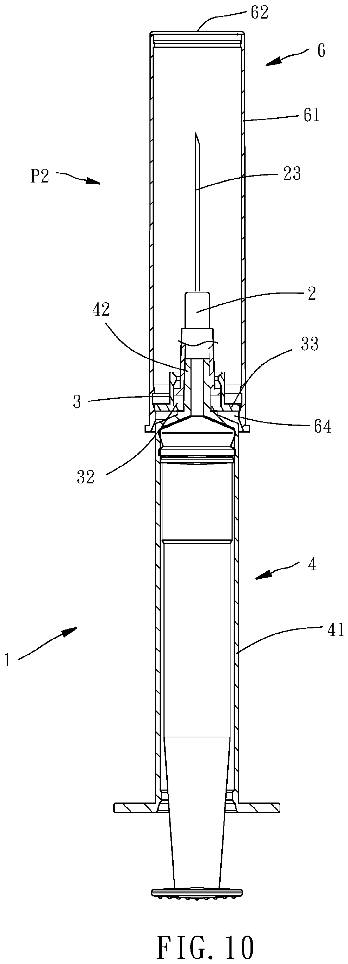

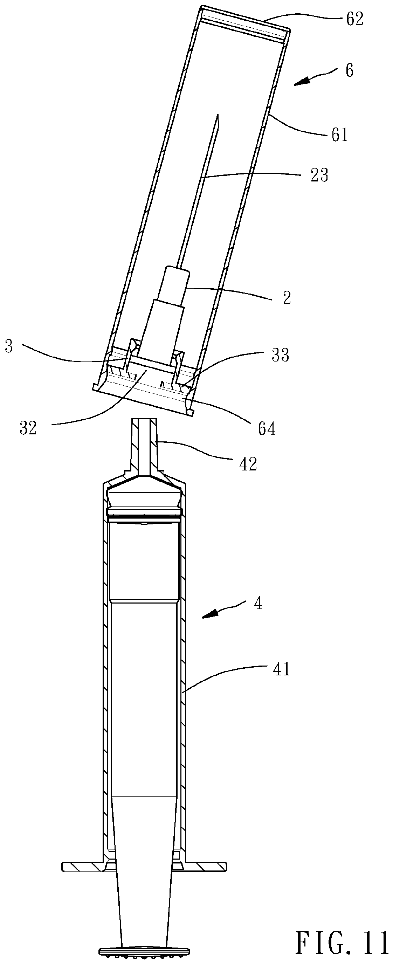

Referring to FIG. 10, after the use of the safety syringe 1 finishes, the outer sleeve 6 is moved frontward to a second position P2. The inner radius of the second tube body 61 of the outer sleeve 6 at the first annular protruding rib 64 is a little larger than the outer radius of the first tube body 41 of the injection barrel 4, and a little smaller than the outer radius of the annular wing portion 33 of the connecting seat 3. Therefore, when the outer sleeve 6 is moved to the second position P2, the first annular protruding rib 64 passes the front end of the injection barrel 4 to be abutted against the annular wing portion 33. At this time, the needle seat 2, the needle 23 and the connecting seat 3 are all accommodated in the second tube body 61. Continuously, the outer sleeve 6 and the injection barrel 4 are forced toward opposite directions. The first annular protruding rib 64 pushes the annular wing portion 33. The injection portion 42 of the injection barrel 4 is separated from the first axial hole 21 of the needle seat 2 and the second axial hole 32 of the connecting seat 3, so that the detachment is accomplished. As shown in FIG. 11, only the needle seat 2 and the connecting seat 3 remain in the outer sleeve 6.

After the outer sleeve 6, the needle seat 2 and the connecting seat 3 are separated from the injection barrel 4, the annular wing portion 33 of the connecting seat 3, because of having the grooves 331, is firmly abutted against the first annular protruding rib 64 of the outer sleeve 6, so that the needle seat 2 is prevented from sliding in the outer sleeve 6 to cause the needle 23 to be exposed out through the first opening 62 to cause danger of pricking.

In addition, the inner circumferential surface of the second tube body 61 of the outer sleeve 6 can be further provided with a second annular protruding rib 66 and a third annular protruding rib 67. The inner radius of the second tube body 61 at the first annular protruding rib 64 is smaller than those at the second annular protruding rib 66 and the third annular protruding rib 67. Therefore, when moving the outer sleeve 6, the user only needs to apply a little force to make the annular wing portion 33 of the connecting seat 3 pass the positions of the second tube body 61 provided with the second annular protruding rib 66 and the third annular protruding rib 67. When the outer sleeve 6 is located at the first position P1, the annular wing portion 33 is located between the second annular protruding rib 66 and the abutted portion 65. When the outer sleeve 6 is located at the second position P2, the annular wing portion 33 is located between the first annular protruding rib 64 and the third annular protruding rib 67. The second annular protruding rib 66 and the third annular protruding rib 67 are auxiliary at the first position P1 and the second position P2 in fixing the annular wing portion 33 to prevent the needle seat 2 from sway.

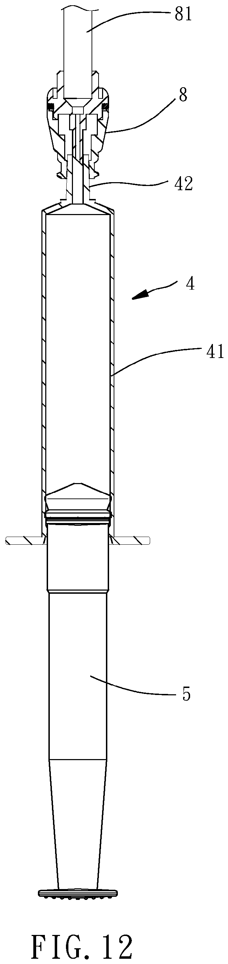

Referring to FIG. 12, the safety syringe 1 can be used in coordination with a needle-free medicine adding connector 8. After medicine is drawn into the injection barrel 4, the outer sleeve 6 can be moved to the second position P2 for the outer sleeve 6, the needle seat 2 and the connecting seat 3 to be removed. After that, the injection portion 42 is inserted into the needle-free medicine adding connector 8, and then the plunger 5 is pushed to make the medicine flow into an infusion pipe 81 through the needle-free medicine adding connector 8. Because the needle 23 has been removed, the situation that the infusion pipe 81 is accidentally pierced by the needle 23 inserted into the needle-free medicine adding connector 8 can be avoided.

It can be known from the above description of the embodiment that the present invention has the following advantages when compared with the existing technique.
1. The safety syringe of the present invention uses the connecting seat and the outer sleeve to provide a design for the separation of the needle seat from the injection barrel and the complete protection for the needle to be discarded, thereby preventing healthcare workers from being pricked by the used needle, improving the safety of the syringe. The retained injection barrel can be used continuously to reduce material waste and effectively lower medical costs.
2. The safety syringe of the present invention includes the connecting seat having the annular wing portion provided with a plurality of grooves for increasing hardness for the annular wing portion, resulting in that when healthcare workers perform injection or blood collection, the needle seat is relatively more stable without sway caused by the elastic deformation of the annular wing portion, so that the usage safety and the operational convenience are increased.

## Claims

1. A safety syringe (1), the safety syringe (1) being **characterized in** comprising:
a needle seat (2) provided at a rear end thereof with a first axial hole (21);
a connecting seat (3) having an annular wall portion (31), a second axial hole (32) penetrating through two ends of the annular wall portion (31), an annular wing portion (33) provided on an outer circumferential surface of a rear end of the annular wall portion (31), and a stopping portion (34) provided on an inner circumferential surface of the rear end of the annular wall portion (31), the annular wall portion (31) being sleeved onto the rear end of the needle seat (2), the second axial hole (32) communicating with the first axial hole (21) of the needle seat (2), the annular wing portion (33) being formed with a plurality of grooves (331) arranged equidistantly;
an injection barrel (4) having a first tube body (41), an injection portion (42) integrally formed at a front end of the first tube body (41), and an accommodating opening (43) provided at a rear end of the first tube body (41), the injection portion (42) being detachably inserted in the first axial hole (21) of the needle seat (2) and the second axial hole (32) of the connecting seat (3);
a plunger (5) movably inserted in the first tube body (41) of the injection barrel (4); and
an outer sleeve (6) having a second tube body (61), a first opening (62) provided at a front end of the second tube body (61), a second opening (63) provided at a rear end of the second tube body (61), a first annular protruding rib (64) located adjacent to the second opening (63), and an abutted portion (65) provided at the front end of the second tube body (61), the first annular protruding rib (64) being provided on an inner circumferential surface of the second tube body (61), the abutted portion (65) extending from the first opening (62) inwardly, the outer sleeve (6) being sleeved onto the injection barrel (4) and movable between a first position (P1) and a second position (P2) along an axial direction of the injection barrel (4);
wherein when the outer sleeve (6) is located at the first position (P 1), the second tube body (61) of the outer sleeve (6) is sleeved onto the first tube body (41) of the injection barrel (4), the needle seat (2) protrudes out of the first opening (62) of the outer sleeve (6), and the abutted portion (65) is abutted against the annular wing portion (33) of the connecting seat (3); when the outer sleeve (6) is located at the second position (P2), the needle seat (2) and the connecting seat (3) are accommodated in the second tube body (61) of the outer sleeve (6), and the first annular protruding rib (64) is enabled to push the annular wing portion (33) of the connecting seat (3) such that the outer sleeve (6), the needle seat (2) and the connecting seat (3) are separated from the injection barrel (4) together.

2. The safety syringe (1) as claimed in claim 1, the safety syringe (1) being **characterized in that** an outer circumferential surface of the rear end of the needle seat (2) has an outer flange (22).

3. The safety syringe (1) as claimed in claim 2, the safety syringe (1) being **characterized in that** an inner circumferential surface of the annular wall portion (31) of the connecting seat (3) has an inner flange (35); the outer flange (22) of the needle seat (2) and the inner flange (35) of the connecting seat (3) are abutted against each other.

4. The safety syringe (1) as claimed in claim 2, the safety syringe (1) being **characterized in that** the inner circumferential surface of the annular wall portion (31) of the connecting seat (3) has an internal thread (36); the outer flange (22) of the needle seat (2) and the internal thread (36) of the connecting seat (3) are engaged with each other.

5. The safety syringe (1) as claimed in claim 1, the safety syringe (1) being **characterized in that** the stopping portion (34) of the connecting seat (3) comprises a plurality of positionally limiting protrusions (342) arranged equidistantly; the front end of the first tube body (41) of the injection barrel (4) is formed with a plurality of positioning protrusions (44) arranged equidistantly; when the injection portion (42) is inserted in the first axial hole (21) of the needle seat (2) and the second axial hole (32) of the connecting seat (3), the plurality of positioning protrusions (44) are jammed in gaps (343) between the plurality of positionally limiting protrusions (342).

6. The safety syringe (1) as claimed in claim 1, the safety syringe (1) being **characterized in that** an outer circumferential surface of the annular wall portion (31) of the connecting seat (3) has a plurality of longitudinal outer ribs (311).

7. The safety syringe (1) as claimed in claim 1, the safety syringe (1) being **characterized in that** an outer circumferential surface of the first tube body (41) of the injection barrel (4) has a plurality of longitudinal ribs (411); when the outer sleeve (6) is located at the first position (P1), the first annular protruding rib (64) of the outer sleeve (6) is tightly abutted against the plurality of longitudinal ribs (411) of the injection barrel (4).

8. The safety syringe (1) as claimed in claim 1, the safety syringe (1) being **characterized in that** the inner circumferential surface of the second tube body (61) of the outer sleeve (6) further has a second annular protruding rib (66) and a third annular protruding rib (67); the second annular protruding rib (66) is provided adjacent to the first opening (62); the third annular protruding rib (67) is provided adjacent to the first annular protruding rib (64); when the outer sleeve (6) is located at the first position (P1), the annular wing portion (33) of the connecting seat (3) is located between the second annular protruding rib (66) and the abutted portion (65) of the outer sleeve (6); when the outer sleeve (6) is located at the second position (P2), the annular wing portion (33) of the connecting seat (3) is located between the first annular protruding rib (64) and the third annular protruding rib (67).
